# FASCICULE DE BREVET EUROPEEN

(11) **EP 2 998 782 B1**
(45) Date de publication et mention de la délivrance du brevet: **15.02.2017**
(21) Numéro de dépôt: 15186146.5
(22) Date de dépôt: 22.09.2015
(51) Int. Cl.: G02B 27/22, A61F 9/02, G02B 27/01, H04N 13/04

(54) **SYSTEME DE VISUALISATION STEREOSCOPIQUE MONOCHROME A PROJECTION SUR LAME SEMI-TRANSPARENTE**
MONOCHROMES STEREO-BETRACHTUNGSSYSTEM MIT PROJEKTION AUF HALB-TRANSPARENTE BLENDE
MONOCHROME STEREOSCOPIC VIEWING SYSTEM WITH PROJECTION ON SEMI-TRANSPARENT SLIDE

(30) Priorité: 22.09.2014 FR 1402109
(43) Date de publication de la demande: 23.03.2016
(73) Titulaire: THALES, 92400 Courbevoie (FR)
(72) Inventeur: CONI, Philippe, 33127 ST JEAN D'ILLAC (FR); LALUQUE, Laurent, 33000 BORDEAUX (FR); GUEGUEN, Aude, 33187 LE HAILLAN Cedex (FR)
(74) Mandataire: Bréda, Jean-Marc

(56) Documents cités:
- WO-A1-2005/039192
- US-A1- 2006 098 093
- US-B1- 6 283 597
- Patrick Murphy: "International Laser Display Association Lasers and Aviation Safety Lasers and Aviation Safety Introduction: Lasers in Airspace", , 10 septembre 2009 (2009-09-10), XP055241121, Extrait de l'Internet: URL:http://www.laserist.org/files/Lasers-a nd-aviation-safety_2pt2.pdf [extrait le 2016-01-13]

## Description

Le domaine de l'invention est celui des systèmes de visualisation permettant de présenter une image en superposition sur le mode extérieur. Les applications techniques sont principalement l'aide à la conduite de véhicule. L'invention s'applique tout particulièrement au domaine des planches de bord d'aéronef où le pilote a besoin de voir simultanément l'extérieur et d'avoir des informations sur la conduite de vol ou la navigation de l'appareil. L'invention peut aussi s'appliquer à tous types de systèmes de conduite et de contrôle affichant des symboles en superposition sur un environnement extérieur naturel. C'est le cas, par exemple, des tours de contrôle ou des postes de navigation de navire. L'environnement extérieur peut également être simulé. C'est, le cas, des simulateurs de pilotage ou des plateformes de contrôle et de commandement des drones.

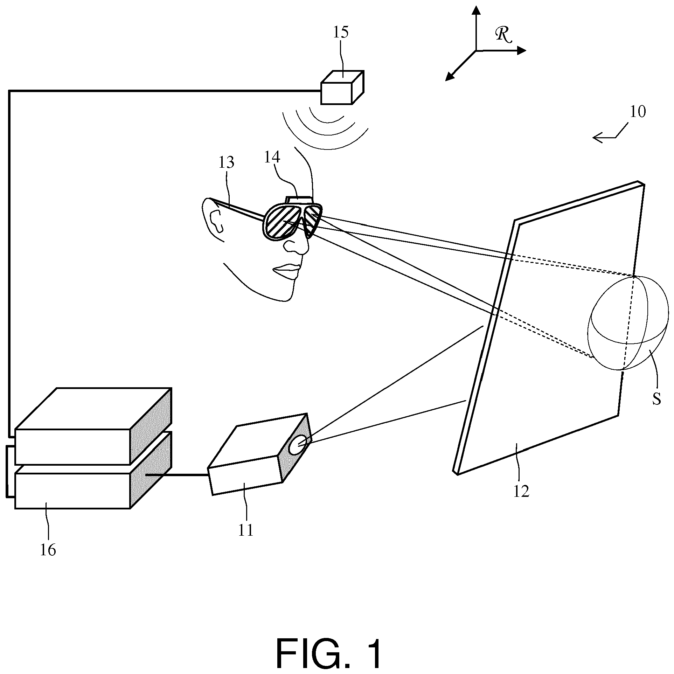

Il existe différents types de systèmes de visualisation permettant d'assurer la superposition d'une image synthétique sur un environnement extérieur. Une solution possible exposée en figure 1 consiste à mettre en oeuvre un projecteur d'images stéréoscopique. Le système de visualisation 10 comprend alors :
- Un projecteur 11 d'images stéréoscopiques dites « 3D » capable de générer au moins deux images appelées « OEil Droit » / « OEil Gauche » représentatives d'un même objet. Dans le cas de la figure 1, l'objet est une sphère S ;
- Un écran 12 semi-transparent diffusant sur lequel sont projetées les images « OEil Droit » / « OEil Gauche » ;
- Une paire de lunettes 13 comportant des moyens de séparation des images « OEil Droit » / « OEil Gauche » et des premiers moyens de détection 14 et destinée à être portée par un utilisateur ;
- des seconds moyens de détection 15 liés à un repère fixe 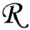 et qui, associés aux premiers moyens de détection 14 permettent la détection de la position spatiale de la paire de lunettes 13 dans ce repère fixe ;
- Un calculateur électronique 16 comprenant au moins les fonctions suivantes :
   o Acquisition des signaux issus des moyens de détection 14 et /ou 15 et calcul de la position de la paire de lunettes ;
   o Calcul de la position de l'image stéréoscopique correspondant à la position de la paire de lunettes ;
   o Calcul des deux images OEil Droit / OEil Gauche.

Il existe différents moyens d'assurer la séparation stéréoscopique des images projetées.

Dans une première solution technique, on utilise la séparation temporelle. Le projecteur envoie séquentiellement et de façon synchronisée d'abord l'image OEil Droit puis l'image OEil Gauche. Les lunettes sont actives et comportent des « shutters » ou occultants actifs synchronisés avec le projecteur. Ainsi, chaque oeil perçoit l'image qui lui est destiné et uniquement celle-ci. Les shutters sont généralement réalisés à base de technologie à cristaux liquides. Cette solution présente plusieurs inconvénients.

Les lunettes actives nécessitent une alimentation et une électronique de commande, ce qui pose des problèmes de maintenance dans le cadre d'une utilisation embarquée. De plus, les polariseurs des shutters LCD provoquent un obscurcissement des visualisations de cockpit, pouvant aller jusqu'à l'occultation totale, en fonction des différentes directions de polarisation et de l'inclinaison des lunettes. Enfin, la présence de polariseurs et l'alternance de vision gauche-droite nécessaire à la vision stéréoscopique entraîne une perte importante de la quantité de lumière. La transmission des lunettes ne dépasse alors pas 30 %, ce qui provoque un obscurcissement rédhibitoire du paysage extérieur.

Dans une seconde solution technique, le projecteur stéréoscopique fonctionne en mode polarisé. Il émet successivement et périodiquement une image OEil Droit selon une première polarisation et une image OEil Gauche selon une seconde polarisation, différente de la première polarisation. La paire de lunettes 13 et passive. Elle comporte un premier verre polarisé transparent à la première polarisation et opaque à la seconde et un second verre polarisé transparent à la seconde polarisation et opaque à la première.

Les lunettes à polariseur sont passives et résolvent la problématique de l'occultation alternée de chaque oeil, ainsi que la gestion des piles. Par contre, il faut impérativement utiliser un écran de projection argenté qui conserve la polarisation. Cet écran n'étant pas transparent, il ne convient pas aux applications concernées par l'invention.

Dans une troisième solution technique, le projecteur émet deux images colorées dont les spectres d'émission sont séparés. La paire de lunettes comporte deux filtres différents, le premier transmet le premier spectre et filtre le second spectre. Le second filtre assure la fonction inverse. Ainsi, chaque oeil perçoit une et une seule image colorée et uniquement celle-ci. Cette technique est connue sous le nom d'anaglyphe. La façon la plus simple de réaliser un anaglyphe est de séparer le spectre visible en deux parties, l'une rouge et l'autre bleue. L'avantage évident du dispositif est sa grande simplicité de mise en oeuvre, mais la vision du monde extérieur est fortement altérée.

Plus perfectionné, le système dit à multiplexage spectral sépare le spectre visible en deux parties entrelacées, une dédiée à chaque oeil. Mais, si la colorimétrie du paysage est mieux préservée, la luminance est considérablement diminuée. Les demandes de brevet de la société « Dolby Laboratories Licensing Corporation » US 2011/0205494, US 2013/0342904 et US 2014/0022637 décrivent des solutions de ce type pour des applications cinématographiques qui ne nécessitent ni des niveaux de lumière élevée ni, bien entendu, de transmission de paysage extérieur.

Le document US2006098093 décrit un dispositif de visualisation d'images stéréoscopiques comportant un projecteur d'images stéréoscopiques, un écran semi-transparent et une paire de lunettes à filtres colorés.

Le système selon l'invention ne présente pas ces inconvénients. Il repose sur le fait que, pour un certain nombre d'applications, l'emploi d'images colorées n'est pas nécessaire. Dans le domaine de la superposition d'images sur un paysage extérieur, il peut être préférable d'utiliser une symbologie monochrome qui se détachera parfaitement sur le fond extérieur plutôt qu'une image colorée qui risque d'introduire de la confusion sur la perception du paysage. Le système selon l'invention met en oeuvre des images stéréoscopiques monochromes émises à des longueurs d'onde différentes mais suffisamment proches pour donner la même sensation visuelle colorée.

Plus précisément, l'invention a pour objet un système de visualisation comportant des moyens de génération d'images stéréoscopiques d'un objet prédéterminé, un dispositif de visualisation desdites images stéréoscopiques comportant un projecteur d'images stéréoscopiques et un écran semi-transparent et une paire de lunettes stéréoscopiques, les moyens de génération d'images stéréoscopiques, le dispositif de visualisation, l'écran semi-transparent et la paire de lunettes stéréoscopiques étant agencés de façon que l'image stéréoscopique de l'objet prédéterminé apparaisse, à travers les lunettes stéréoscopiques, à une distance prédéterminée de l'écran semi-transparent, caractérisé en ce que :
le projecteur comporte des moyens agencés de façon à projeter alternativement une première image émise à une et une seule première longueur d'onde et une seconde image émise à une et une seule seconde longueur d'onde différente de la première longueur d'onde,
la paire de lunette comporte un premier filtre disposé devant l'oeil droit et un second filtre disposé devant l'oeil gauche, le premier filtre transmettant la totalité du spectre à l'exception d'une première bande spectrale étroite centrée sur la première longueur d'onde et le second filtre transmettant la totalité du spectre à l'exception d'une seconde bande spectrale étroite centrée sur la seconde longueur d'onde.

Avantageusement, la première longueur d'onde est séparée de la seconde longueur d'onde par une distance spectrale de l'ordre de 10 à 30 nanomètres.

Avantageusement, la première longueur d'onde et la seconde longueur d'onde sont situées entre 500 nanomètres et 560 nanomètres.

Avantageusement, la largeur à mi-hauteur de la première bande spectrale et de la seconde bande spectrale est comprise entre 10 nanomètres et 30 nanomètres.

Avantageusement, le projecteur comporte au moins une matrice d'affichage éclairée alternativement par deux sources émettant respectivement à la première longueur d'onde et à la seconde longueur d'onde.

Avantageusement, la paire de lunette comporte un troisième filtre à une des longueurs d'onde émises par les pointeurs laser grand public de classes 3A, 3B et 4.

Avantageusement, la première bande spectrale et la seconde bande spectrale se chevauchent en partie, une des longueurs d'onde émises par les pointeurs laser grand public de classes 3A, 3B et 4 étant située dans ladite zone de chevauchement de façon à être filtrée par le premier filtre et par le second filtre.

Avantageusement, le système comporte des moyens de détection de la position relative de la paire de lunettes par rapport à la position de l'écran semi-transparent et des moyens de calcul des images stéréoscopiques de façon que la position de l'image stéréoscopique de l'objet soit fixe dans un repère prédéterminé et indépendante de la position des lunettes stéréoscopiques.

Avantageusement, la distance prédéterminée est comprise entre quelques centimètres et l'infini optique.

Avantageusement, le projecteur comporte des moyens d'affichage d'une troisième image non stéréoscopique.

Avantageusement, le système de visualisation est un système de cockpit d'aéronef.

L'invention sera mieux comprise et d'autres avantages apparaîtront à la lecture de la description qui va suivre donnée à titre non limitatif et grâce aux figures annexées parmi lesquelles :
La figure 1 représente une architecture d'un système de visualisation selon l'art antérieur ;
La figure 2 représente une architecture d'un système de visualisation selon l'invention ;
La figure 3 représente la transmission du premier filtre des lunettes stéréoscopiques en fonction de la longueur d'onde et l'emplacement des deux longueurs d'onde d'émission ;
La figure 4 représente la transmission du second filtre des lunettes stéréoscopiques en fonction de la longueur d'onde et l'emplacement des deux longueurs d'onde d'émission ;
La figure 5 représente un agencement particulier du premier filtre et du second filtre permettant d'éliminer conjointement une longueur d'onde ou une bande spectrale particulière.

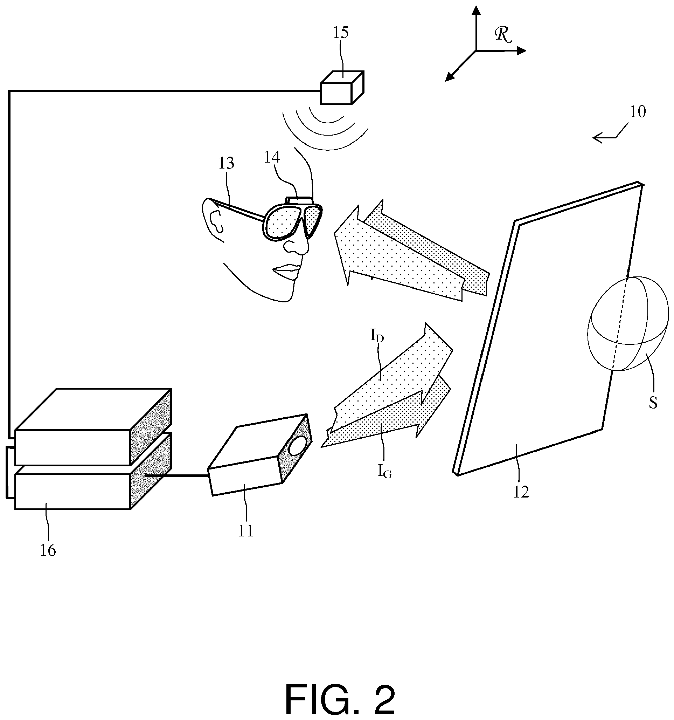

A titre d'exemple, un système de visualisation selon l'invention est représenté en figure 2. Il comporte au moins :
- Un projecteur 11 d'images stéréoscopiques monochromes comportant un afficheur haute résolution et une optique de projection ayant un grandissement suffisant pour couvrir l'écran semi-transparent. Pour les applications aéronautiques, il est important que la luminance maximale de l'afficheur puisse être très élevée ;
- un écran semi-transparent 12. C'est une lame optique possédant à la fois une semi-transparence du paysage extérieur et une diffusion des images stéréoscopiques. A cette fin, la surface de l'écran de projection peut comporter un réseau de motifs diffusants ou « patterns ». La diffusion de l'écran se fait dans un large angle de vue, voisin du demi-espace. On obtient ainsi une boite à oeil de grande dimension. On entend par « boîte à oeil » la zone de l'espace où l'image est visible. Cette solution permet également de maîtriser parfaitement la transparence de l'écran. Ainsi, si les motifs ne couvrent qu'un pourcentage limité de la surface de l'écran, la transmission de l'écran est égale à l'unité moins le pourcentage couvert par les motifs. Par exemple, si les motifs couvrent 20% de la surface, la transmission de l'écran est voisine de 80%. Il est à noter que, dans la mesure où le projecteur émet des images monochromes, il est possible d'adapter le coefficient de réflexion des motifs de façon qu'ils soient parfaitement réfléchissants dans la gamme de longueurs d'onde d'émission et parfaitement transparents en dehors de cette gamme ;
- une paire de lunettes stéréoscopiques à filtres spectraux portée par l'utilisateur.

Le fonctionnement du système de visualisation est le suivant. L'afficheur affiche alternativement deux images stéréoscopiques I_{G} et I_{D} représentant un objet qui est une sphère S sur la figure 2, la première est éclairée à une première longueur d'onde, la seconde image est éclairée à une seconde longueur d'onde. Ces deux longueurs d'onde sont voisines mais différentes. A titre d'exemple, la première longueur d'onde est séparée de la seconde longueur d'onde par une distance spectrale de l'ordre de quelques nanomètres à 30 nanomètres. La première longueur d'onde et la seconde longueur d'onde sont situées entre 500 nanomètres et 560 nanomètres. Par exemple, la première source peut émettre à 520 nanomètres et la seconde source à 540 nanomètres.

On peut facilement trouver des sources d'émission modulables émettant à de telles longueurs d'onde en utilisant, par exemple, des diodes laser ou des diodes électroluminescentes filtrées. On peut également utiliser des sources de lumière blanches filtrées en plusieurs bandes spectrales.

La paire de lunette comporte un premier filtre disposé devant l'oeil droit et un second filtre disposé devant l'oeil gauche, le premier filtre transmettant la totalité du spectre à l'exception d'une première bande spectrale étroite centrée sur la première longueur d'onde et le second filtre transmettant la totalité du spectre à l'exception d'une seconde bande spectrale étroite centrée sur la seconde longueur d'onde, la première bande spectrale et la seconde bande spectrale ne se chevauchant pas.

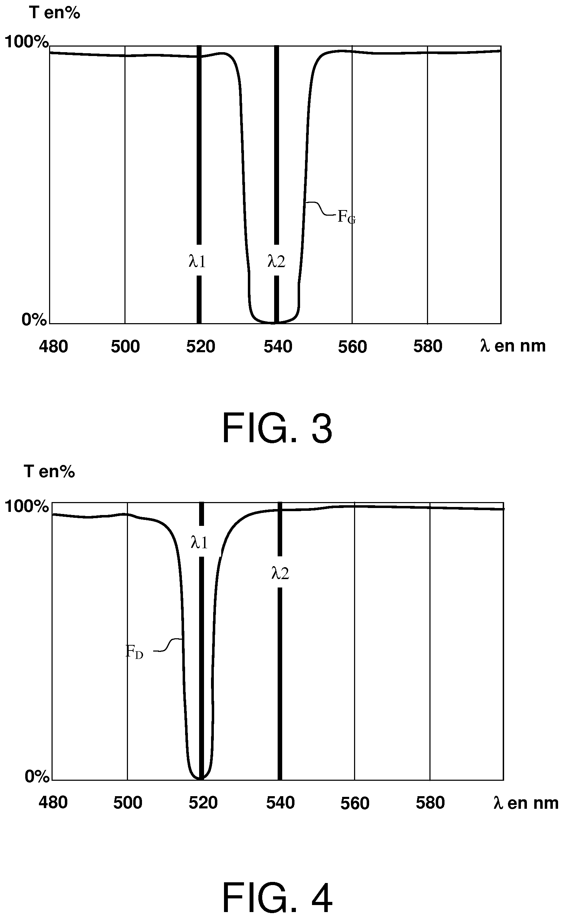

A titre d'exemple, les figures 3 et 4 représentent la transmission du premier filtre F_{G} et du second filtre F_{D} en fonction de la longueur d'onde et l'emplacement des deux longueurs d'onde d'émission λ1 et λ2 qui sont situées respectivement à 520 et 540 nanomètres. Le premier filtre F_{G} transmet la première longueur d'onde et coupe totalement la seconde. Le second filtre F_{D} transmet la seconde longueur d'onde et coupe totalement la première. La réalisation de tels filtres optiques ne présente pas de difficultés particulières. Ils sont connus sous l'appellation anglo-saxonne de filtres « notch ».

Ainsi, l'oeil gauche ne peut voir que l'image émise à la première longueur d'onde et l'oeil droit ne peut voir que l'image émise à la seconde longueur d'onde. Les images stéréoscopiques successives sont bien séparées et l'illusion stéréoscopique est restituée. L'utilisateur perçoit une image fusionnée virtuellement placée à une certaine distance de l'écran de visualisation, cette distance pouvant être l'infini pour certaines applications. L'objet peut être bidimensionnel s'il s'agit, par exemple, d'un symbole ou tridimensionnel.

Le système permet alors de générer des objets dans une très large gamme de distances allant de l'infini à des distances très proches de l'utilisateur. Ainsi, l'image stéréoscopique peut représenter un objet placé devant l'écran semi-transparent.

Un des avantages important de ce système est que la paire de lunettes stéréoscopiques a une excellente transmission à la différence des systèmes de l'art antérieur. En effet, seule une bande spectrale étroite du paysage extérieur est bloquée par les filtres.

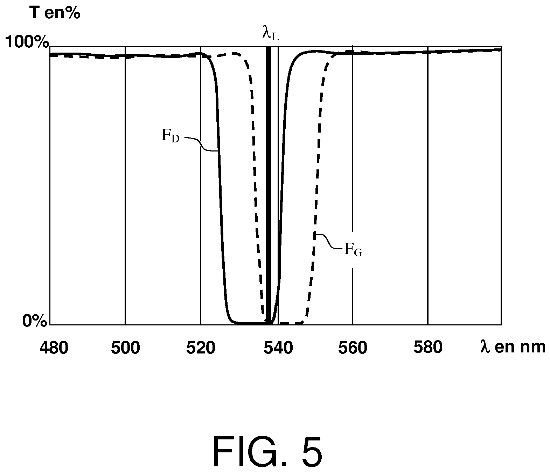

Un autre avantage est que l'on peut filtrer une des longueurs d'onde émises par les pointeurs laser grand public de classes 3A, 3B et 4 de façon à protéger l'utilisateur de ces pointeurs. Une solution possible est d'ajouter aux filtres du système stéréoscopique un filtre supplémentaire qui bloque cette longueur d'onde. Une autre solution possible est illustrée sur la figure 5. La première bande spectrale du premier filtre F_{G} et la seconde bande spectrale du second filtre F_{D} se chevauchent en partie, ladite longueur d'onde émise λ_{L} par un pointeur laser grand public étant située dans ladite zone de chevauchement de façon à être filtrée par le premier filtre et par le second filtre. Avec cette dernière solution, il n'est pas nécessaire d'ajouter de filtres spécifiques.

Le système selon l'invention peut comporter un système de détection de la position de la paire de lunettes. Ce type de détection comporte classiquement deux sous-ensembles, comme on le voit sur la figure 2, un premier sous-ensemble 14 fixé à la paire de lunettes, un second sous-ensemble 15 disposé dans un repère fixe. Il existe différentes techniques permettant de repérer un objet dans l'espace. On peut utiliser la détection électromagnétique. Un émetteur est disposé dans le repère fixe et un récepteur dans le repère mobile. On peut également utiliser la détection optique qui peut être passive ou active. Dans ce dernier cas, la paire de lunettes porte des diodes électroluminescentes dont la position de l'émission est repérée par des caméras. Toutes ces techniques sont connues de l'homme du métier. Elles sont compatibles d'un fonctionnement en temps réel et s'adaptent facilement au système de visualisation selon l'invention.

Lorsque l'utilisateur bouge la tête, ses mouvements sont captés par les moyens de détection de la paire de lunette. Le calculateur électronique recalcule alors en temps réel la position des images stéréoscopiques de façon que l'utilisateur continue de voir l'image virtuelle de l'objet à la même place. Pour prendre un exemple simple, si l'image virtuelle de l'objet est à l'infini, les images stéréoscopiques OEil Droit et OEil Gauche sont séparées d'une distance qui vaut sensiblement la distance moyenne interpupillaire d'un être humain. Leur déplacement sur l'écran de visualisation est sensiblement égal à celui de la paire de lunette. On crée ainsi la sensation d'image à l'infini.

Le système de visualisation selon l'invention permet également de générer facilement des images en couleur non stéréoscopiques. Il suffit que ces images soient émises dans une bande spectrale rouge, une bande spectrale bleue et une troisième bande spectrale verte suffisamment large pour être transmise par les deux filtres de la paire de lunettes stéréoscopique de façon à ne pas perturber la vision colorée.

Les applications techniques du système de visualisation selon l'invention sont principalement l'aide à la conduite de véhicule. Le système selon l'invention s'applique tout particulièrement au domaine des planches de bord d'aéronef où le pilote a besoin à la fois de voir l'extérieur et d'avoir des informations sur la conduite de vol ou la navigation de l'appareil. L'application dans le domaine des hélicoptères est particulièrement intéressante dans la mesure où les hélicoptères possèdent des verrières de grande dimension et sont amenés à effectuer des vols à basse altitude.

## Revendications

1. Système de visualisation (10) comportant des moyens (16) de génération d'images stéréoscopiques d'un objet prédéterminé, un dispositif de visualisation desdites images stéréoscopiques comportant un projecteur (11) d'images stéréoscopiques et un écran semi-transparent (12) et une paire de lunettes stéréoscopiques (13), les moyens (16) de génération d'images stéréoscopiques, le dispositif de visualisation (11), l'écran semi-transparent (12) et la paire de lunettes (13) stéréoscopiques étant agencés de façon que l'image stéréoscopique de l'objet prédéterminé apparaisse, à travers les lunettes stéréoscopiques, à une distance prédéterminée de l'écran semi-transparent,
**caractérisé en ce que**
le projecteur comporte des moyens agencés de façon à projeter alternativement une première image émise à une et une seule première longueur d'onde et une seconde image émise à une et une seule seconde longueur d'onde différente de la première longueur d'onde,
la paire de lunette comporte :
un premier filtre disposé devant l'oeil droit et un second filtre disposé devant l'oeil gauche, le premier filtre transmettant la totalité du spectre à l'exception d'une première bande spectrale étroite centrée sur la première longueur d'onde et le second filtre transmettant la totalité du spectre à l'exception d'une seconde bande spectrale étroite centrée sur la seconde longueur d'onde,
des moyens de filtrage à une des longueurs d'onde (λ_{L}) émises par les pointeurs laser grand public de classes 3A, 3B et 4.

2. Système de visualisation selon la revendication 1, **caractérisé en ce que** la première bande spectrale et la seconde bande spectrale se chevauchent en partie, une des longueurs d'onde émises par les pointeurs laser grand public de classes 3A, 3B et 4 étant située dans ladite zone de chevauchement de façon à être filtrée par le premier filtre et par le second filtre.

3. Système de visualisation selon l'une des revendications précédentes, **caractérisé en ce que** la première longueur d'onde est séparée de la seconde longueur d'onde par une distance spectrale de l'ordre de 10 à 30 nanomètres.

4. Système de visualisation selon l'une des revendications précédentes, **caractérisé en ce que** la première longueur d'onde et la seconde longueur d'onde sont situées entre 500 nanomètres et 560 nanomètres.

5. Système de visualisation selon l'une des revendications précédentes, **caractérisé en ce que** la largeur à mi-hauteur de la première bande spectrale et de la seconde bande spectrale est comprise entre 10 nanomètres et 30 nanomètres.

6. Système de visualisation selon l'une des revendications précédentes, **caractérisé en ce que** le projecteur comporte au moins une matrice d'affichage éclairée alternativement par deux sources émettant respectivement à la première longueur d'onde et à la seconde longueur d'onde.

7. Système de visualisation selon l'une des revendications précédentes, **caractérisé en ce que** le système comporte des moyens de détection (14, 15) de la position relative de la paire de lunettes par rapport à la position de l'écran semi-transparent et des moyens de calcul des images stéréoscopiques de façon que la position de l'image stéréoscopique de l'objet soit fixe dans un repère prédéterminé et indépendante de la position des lunettes stéréoscopiques.

8. Système de visualisation selon l'une des revendications précédentes, **caractérisé en ce que** la distance prédéterminée est comprise entre quelques centimètres et l'infini optique.

9. Système de visualisation selon l'une des revendications précédentes, **caractérisé en ce que** le projecteur comporte des moyens d'affichage d'une troisième image non stéréoscopique.

10. Système de visualisation selon l'une des revendications précédentes, **caractérisé en ce que** le système de visualisation est un système de cockpit d'aéronef.

## Patentansprüche

1. Visualisierungssystem (10), umfassend Mittel (16) zum Erzeugen von stereoskopischen Bildern eines vorbestimmten Objekts, eine Vorrichtung zum Visualisieren der stereoskopischen Bilder, die einen stereoskopischen Bildprojektor (11) und einen teiltransparenten Bildschirm (12) und eine stereoskopische Brille (13) umfasst, wobei die Mittel (16) zum Erzeugen von stereoskopischen Bildern, die Visualisierungsvorrichtung (11), der teiltransparente Bildschirm (12) und die stereoskopische Brille (13) so ausgelegt sind, dass das stereoskopische Bild des vorbestimmten Objekts durch die stereoskopische Brille in einem vorbestimmten Abstand von dem teiltransparenten Bildschirm erscheint,
**dadurch gekennzeichnet, dass**
der Projektor Mittel umfasst, die so angeordnet sind, dass sie abwechselnd ein mit nur einer einzigen ersten Wellenlänge emittiertes erstes Bild und ein mit nur einer einzigen zweiten Wellenlänge, die sich von der ersten Wellenlänge unterscheidet, emittiertes zweites Bild projizieren,
die Brille Folgendes umfasst:
ein erstes Filter, das vor dem rechten Auge angeordnet ist, und ein zweites Filter, das vor dem linken Auge angeordnet ist, wobei das erste Filter das volle Spektrum durchlässt, mit Ausnahme eines ersten schmalen Spektralbandes, das auf der ersten Wellenlänge zentriert ist, und wobei das zweite Filter das volle Spektrum emittiert, mit Ausnahme eines zweiten schmalen Spektralbandes, das auf der zweiten Wellenlänge zentriert ist,
Mittel zum Filtern bei einer der Wellenlängen (λ_{L}), die von Massenprodukt-Laserzeigern der Klassen 3A, 3B und 4 emittiert werden.

2. Visualisierungssystem nach Anspruch 1, **dadurch gekennzeichnet, dass** das erste Spektralband und das zweite Spektralband einander teilweise überlappen, wobei eine der von den Massenprodukt-Laserzeigern der Klassen 3A, 3B und 4 erzeugten Wellenlängen in der Überlappungszone liegt, um vom ersten Filter und vom zweiten Filter gefiltert zu werden.

3. Visualisierungssystem nach einem der vorherigen Ansprüche, **dadurch gekennzeichnet, dass** die erste Wellenlänge von der zweiten Wellenlänge um eine spektrale Distanz von etwa 10 bis 30 Nanometern getrennt ist.

4. Visualisierungssystem nach einem der vorherigen Ansprüche, **dadurch gekennzeichnet, dass** die erste Wellenlänge und die zweite Wellenlänge zwischen 500 Nanometern und 560 Nanometern liegen.

5. Visualisierungssystem nach einem der vorherigen Ansprüche, **dadurch gekennzeichnet, dass** die Breite in der mittleren Höhe des ersten Spektralbands und des zweiten Spektralbands zwischen 10 Nanometern und 30 Nanometern liegt.

6. Visualisierungssystem nach einem der vorherigen Ansprüche, **dadurch gekennzeichnet, dass** der Projektor wenigstens eine Anzeigematrix umfasst, die abwechselnd von zwei Quellen beleuchtet wird, die jeweils mit der ersten Wellenlänge und der zweiten Wellenlänge emittieren.

7. Visualisierungssystem nach einem der vorherigen Ansprüche, **dadurch gekennzeichnet, dass** das System Mittel (14, 15) zum Erkennen der relativen Position der Brille mit Bezug auf die Position des teiltransparenten Bildschirms und Mittel zum Berechnen von stereoskopischen Bildern umfasst, so dass die Position des stereoskopischen Bildes des Objekts in einem vorbestimmten Referenzsystem fixiert und von der Position der stereoskopischen Brille unabhängig ist.

8. Visualisierungssystem nach einem der vorherigen Ansprüche, **dadurch gekennzeichnet, dass** die vorbestimmte Distanz zwischen mehreren Zentimetern und optisch unendlich liegt.

9. Visualisierungssystem nach einem der vorherigen Ansprüche, **dadurch gekennzeichnet, dass** der Projektor Mittel zum Anzeigen eines nicht stereoskopischen dritten Bildes umfasst.

10. Visualisierungssystem nach einem der vorherigen Ansprüche, **dadurch gekennzeichnet, dass** das Visualisierungssystem ein Luftfahrzeug-Cockpit-System ist.

## Claims

1. A viewing system (10) comprising means (16) for generating stereoscopic images of a predetermined object, a device for viewing said stereoscopic images comprising a stereoscopic image projector (11) and a semi-transparent screen (12) and a pair of stereoscopic glasses (13), said means (16) for generating stereoscopic images, said viewing device (11), said semi-transparent screen (12) and said pair of stereoscopic glasses (13) being arranged so that the stereoscopic image of said predetermined object appears, through said stereoscopic glasses, at a predetermined distance from said semi-transparent screen,
**characterised in that**
said projector comprises means that are arranged so as to alternately project a first image emitted at strictly one first wavelength and a second image emitted at strictly one second wavelength different from said first wavelength,
said pair of glasses comprises:
a first filter arranged in front of the right eye and a second filter arranged in front of the left eye, said first filter transmitting the full spectrum, except for a first narrow spectral band centred on said first wavelength, and said second filter emitting the full spectrum, except for a second narrow spectral band centred on said second wavelength,
means for filtering at one of the wavelengths (λ_{L}) emitted by mass-market laser pointers of classes 3A, 3B and 4.

2. The viewing system according to claim 1, **characterised in that** said first spectral band and said second spectral band partly overlap, one of the wavelengths emitted by said mass-market laser pointers of classes 3A, 3B and 4 being located in said overlapping zone so as to be filtered by said first filter and by said second filter.

3. The viewing system according to any one of the preceding claims, **characterised in that** said first wavelength is separated from said second wavelength by a spectral distance of approximately 10 to 30 nanometres.

4. The viewing system according to any one of the preceding claims, **characterised in that** said first wavelength and said second wavelength are located between 500 nanometres and 560 nanometres.

5. The viewing system according to any one of the preceding claims, **characterised in that** the width at the mid-height of said first spectral band and said second spectral band is between 10 nanometres and 30 nanometres.

6. The viewing system according to any one of the preceding claims, **characterised in that** said projector comprises at least one display matrix alternately illuminated by two sources respectively emitting at said first wavelength and at said second wavelength.

7. The viewing system according to any one of the preceding claims, **characterised in that** said system comprises means (14, 15) for detecting the relative position of said pair of glasses in relation to the position of said semi-transparent screen and means for computing stereoscopic images so that the position of the stereoscopic image of said object is fixed in a predetermined coordinate system and is independent of the position of said stereoscopic glasses.

8. The viewing system according to any one of the preceding claims, **characterised in that** said predetermined distance is between several centimetres and optical infinity.

9. The viewing system according to any one of the preceding claims, **characterised in that** said projector comprises means for displaying a third non-stereoscopic image.

10. The viewing system according to any one of the preceding claims, **characterised in that** said viewing system is an aircraft cockpit system.
